# EUROPEAN PATENT APPLICATION

(11) **EP 1 547 648 A1**
(43) Date of publication of application: **29.06.2005**
(21) Application number: 04258080.3
(22) Date of filing: 23.12.2004
(51) Int. Cl.: A61N 1/08, A61N 1/362, A61N 1/365, A61N 1/368, A61B 5/04

(54) **System and method for determining optimal pacing sites based on myocardial activation times**

(30) Priority: 24.12.2003 US 745841
(71) Applicant: Pacesetter, Inc., Sylmar, CA 91342-9221 (US)
(72) Inventor: Xiaoyi, Min, Thousand Oaks CA 91362 (US); Yang, Michael, Thousand Oaks CA 91362 (US); Morgan, Kevin L., Simi Valley CA 93063 (US); Falkenberg, Eric, Simi Valley CA 93065 (US)
(74) Representative: Rees, David Christopher

(57) **Abstract**

The optimal location for delivering pacing pulses to a given cardiac chamber (such as the left ventricle) is determined by identifying the last electrically or mechanically activated site within the chamber. For example, the last site within the left ventricular myocardium to depolarize during an intrinsic ventricular contraction triggered by an initial atrial depolarization is identified as being the optimal pacing location for the left ventricle. A pacing electrode is then implanted as close as possible to that location. The technique is particularly effective for use in identifying optimal epicardial pacing locations for mounting satellite pacing devices for use in delivering cardiac resynchronization therapy. However, the techniques may also be applied to identify locations for mounting endocardial electrodes as well. Capture thresholds may also be taken into account in determining the optimal location. Examples are described for both master/satellite pacing systems as well as biventricular single device systems.

## Description

The invention relates generally to implantable cardiac stimulation systems for use in pacing the heart and in particular to techniques for determining optimal locations for mounting one or more electrodes for use primarily in heart failure patients.

Heart failure is one of the most widespread and devastating cardiac afflictions, currently affecting approximately 15 million people worldwide, including over 5 million in the United States. In the U.S., approximately 450,000 new patients are diagnosed with heart failure each year and the majority dies within five years of diagnosis. One factor that contributes to heart failure is asynchronous activation of the ventricles such that the mechanical contraction is not coordinated effectively thus compromising cardiac function. As a result, the pumping ability of the heart is diminished and the patient experiences shortness of breath, fatigue, swelling, and other debilitating symptoms. The weakened heart is also susceptible to potentially lethal ventricular tachyarrhythmias. A decrease in cardiac function can result in a progression of heart failure. In many cases, pacing control parameters of the pacemaker or implantable cardioverter defibrillator (ICD) can be adjusted to help improve cardiac function and reduce the degree of heart failure effectively reducing symptoms and improving the quality of life.

One particularly promising technique for reducing the risk of heart failure is cardiac resynchronization therapy (CRT), which seeks to normalize asynchronous cardiac electrical activation and the resultant asynchronous contractions by delivering synchronized pacing stimulus to both ventricles using pacemakers or implantable cardioverter defibrillators (ICDs) equipped with biventricular pacing capability. The stimulus is synchronized so as to help to improve overall cardiac function. This may have the additional beneficial effect of reducing the susceptibility to life-threatening tachyarrhythmias.

For example, within the patients subject to left bundle branch block, pacing signals delivered to the left ventricle (LV) are timed relative to right ventricular pacing signals for the purpose of improving cardiac function. Briefly, within such patients, natural electrical signal nerve conduction pathways are damaged or blocked and so intrinsic pacing signals from the sinus node do not follow normal pathways through the left bundle branch and into the left ventricular myocardium to allow the left ventricle to contract efficiently and uniformly. Rather, the electrical signals propagate through alternate pathways, particularly through the myocardium itself, resulting in different portions of the left ventricular myocardium contracting at different times. Since the left ventricle does not contract uniformly, its pumping efficacy is reduced and overall cardiac function is impaired. With CRT, pacing pulses are delivered directly to the left ventricle in an attempt to ensure that the left ventricular myocardium will contract more uniformly. A time delay relative to atrial pacing pulses and to right ventricular pacing pulses is set in an attempt to achieve optimal cardiac function. Typically, an RV-LV delay is initially set to zero while an atrioventricular (AV) delay (i.e. the pacing time delay between the atrial and the ventricles) is adjusted to yield the best cardiac function. Then, the RV-LV delay is adjusted to achieve still further improvements in cardiac function. Within most patients, the RV-LV delay is set to a positive value, i.e. the left ventricle is paced slightly before the right ventricle (RV). In other patients, the RV-LV delay is negative such that the right ventricle is paced slightly before the left ventricle. Similar techniques are also employed for patients whose nerve conduction pathways are corrupted due to right bundle branch block or due to other problems such as the development of scar tissue within the myocardium following a myocardial infarction.

Hence, with current state-of-the-art CRT techniques, the relative timing between left ventricular and right ventricular pacing pulses is adjusted in an attempt to improve cardiac function. Although such techniques are effective, it would be desirable to provide further improvements so as to achieve still greater benefits in cardiac function. In particular, whereas current CRT techniques are primarily directed to determining the optimal time delay between various pacing pulses, even greater potential improvement in overall cardiac function may be gained by also identifying the optimal pacing locations for use in conjunction with CRT techniques.

Heretofore, techniques for attempting to identify optimal pacing locations, particularly for use in the ventricles, have typically employed a pacing probe that is repositioned at various locations on or within the ventricles. Test pacing pulses are delivered through the probe and some measure of cardiac function, such as stroke volume, is monitored. A disadvantage of these techniques is that it can be difficult to obtain a reliable measure of cardiac function while pacing at various locations. Also, it would be preferable to employ a simpler sensing probe rather than a more sophisticated pacing probe. An example of a technique for determining electrode placement using a pacing probe while measuring stroke volume is set forth in U.S. Patent Application US2002/0087089 of *Ben-Haim,* entitled "Method of Pacing a Heart Using Implantable Device."

In addition, to the extent that an "optimal" location is identified via predecessor pacing probe techniques, it is typically determined without regard to pacing delay to be employed with CRT. Rather, typically, the AV delay is set to default value and the RV-LV delay is set 0 and then the optimal location is determined based upon those default parameters, with little or no regard to the actual time delay parameters that may need to be employed during actual CRT. Accordingly, the location that is identified as "optimal" may not be optimal once CRT therapy has commenced. In order to properly take into account both CRT time delay values while determining the optimal pacing location, the test probe would need to be repositioned for a wide variety of pacing delay values (both AV delay and RV-LV delay). As far as the inventors are aware, such techniques have not been exploited and would probably be impractical in any case.

Accordingly, it would be highly desirable to provide improved techniques for identifying optimal pacing locations, particularly for use with CRT, that do not require simultaneous measurement of cardiac function and do not necessarily require the use of a test pacing probe.

In accordance with the illustrative embodiments, techniques are provided for determining a preferred or optimal location for pacing within a selected cardiac chamber of a patient. Briefly, information is input for a particular patient pertaining to myocardial activation times within the selected chamber of the patient, which may pertain to electrical activation times or mechanical activation times. The preferred location for pacing within the selected chamber is then determined based on the information pertaining to the myocardial activation times by identifying the last activated site within the myocardium of the selected chamber. The last activated site is the site that that depolarizes or contracts last in response to intrinsic electrical activation signals received from another chamber. A pacing electrode is then positioned at or near the preferred location.

The last activated site is preferred for the following reasons. The last activated site within a given chamber, such as within the left ventricle, corresponds to portions of the myocardium in that chamber that contract last in response to intrinsic pacing pulses and hence most significantly contribute to any uneven contraction of the chamber. By delivering pacing pulses directly at that location, portions of the myocardium that would otherwise contract last during an intrinsic beat can instead be caused to contract sooner -- preferably simultaneously with other portions of the myocardium of that chamber -- thus improving the uniformity of chamber contraction and therefore improving stroke volume from that chamber.

In addition, by identifying the preferred or optimal location based on myocardial activation times, it is believed that the location can be more easily and more reliably identified than with predecessor techniques, such as those requiring that some measure of cardiac function be monitored simultaneously while the heart is paced at various locations. In certain embodiments, myocardial activation times can be determined simply based on electrical signals detected using electrical sensing probes or based on mechanical contraction detected using motion sensing probes, and so there is no need to simultaneously measure cardiac function. Moreover, predecessor techniques typically require the use of a more elaborate pacing probe rather than a simpler sensing probe.

In one specific example, the optimal location for epicardial pacing of the left ventricle using a satellite pacing device is determined by identifying the last electrically activated site within the left ventricular epicardium based on conduction delays from the right atrium. P-waves are detected, either using an electrode mounted within the right atrium (which is connected to a master pacemaker already implanted within the patient) or instead using a surface electrocardiogram (ECG). A sensing probe is placed at various candidate locations on the left ventricular epicardium and corresponding R_{LV}-waves are detected. (Note that, the terms P-wave and R-wave are sometimes used to refer only to features of a surface ECG. Herein, for the sake of brevity and generality, the terms are used to refer to the corresponding electrical events, whether sensed internally or externally.) At each of the various locations, the conduction delay from the right atrium to that location (i.e. PR_{LV}) is measured by detecting the time delay between P-waves and R_{LV}-waves sensed at that location. Alternatively, conduction delays between paced atrial events (A-pulse) and the R_{LV}-waves are detected (i.e. AR_{LV}). In either case, the location having the longest conduction delay corresponds to the last activated site, and that location is identified as the optimal location for epicardial LV pacing. Alternatively, rather than sense depolarization of the left ventricular myocardium using an electrical sensing probe, mechanical contraction of the myocardium can instead be detected using a motion-sensing probe to identify the last activated site. In any case, a contrast agent may be deposited at that location to mark the optimal location for subsequent mounting of an epicardial pacing electrode. The actual determination of optimal pacing locations is preferably preformed by an external programmer device (or other external computing device) based on data received from the sensing probe and from the surface ECG or from data received from the master pacemaker or ICD, if already implanted.

In other examples, the technique is exploited to identify: optimal locations for endocardial pacing in the LV based on AV conduction from the right atria; optimal locations for epicardial or endocardial pacing in the LV based on RV pacing; or optimal locations for epicardial or endocardial pacing in the LV based on time delays between LV pacing (triggered by a pacing probe) and RV sensing. Alternatively, the techniques may be performed to identify optimal pacing locations in the right ventricle or the left atria. Still other examples of the technique take into account capture thresholds in the determination of the optimal location so as to ensure that the selected location does not have a capture threshold that is too high.

Accordingly, improved techniques are provided for identifying preferred or optimal pacing locations.

According to the invention, there is provided a system for determining a preferred location for cardiac pacing for a patient, the system comprising: means for collecting information pertaining to myocardial activation times within a selected heart chamber of the patient; and means for determining the preferred location for pacing within the selected chamber by identifying the last activated site within the myocardium of the selected chamber.

The invention also extends to a method for determining a preferred location for delivering cardiac pacing pulses to the myocardium of a heart chamber of a patient, the method comprising: collecting information pertaining to myocardial activation times within the myocardium of a selected heart chamber of the patient; determining the preferred location for pacing for the selected chamber based on the information pertaining to myocardial activation times by identifying the last activated site within the myocardium of the selected chamber; and positioning one or more electrodes at a location selected based on the preferred location.

Determining the preferred location based on myocardial activation times is preferably performed based on electrical myocardial activation times. The electrical activation times may be determined using an electrical sensing probe. Preferably, determining the preferred location based on myocardial activation times is performed based on mechanical myocardial activation times.

The method may further comprise inputting information for the patient pertaining to capture thresholds for various locations of the myocardium of the selected heart chamber of the patient and the step of determining the preferred location for pacing of the selected chamber may incorporate capture thresholds as well as myocardial activation times.

Determining the preferred location preferably comprises identifying the last site in the myocardium of the selected chamber that is activated due to activation signals originating from another chamber. Preferably, the selected chamber is a selected ventricle and identifying the last activated site preferably comprises determining conduction delays from the right atrium to a plurality of locations within the myocardium of the selected ventricle. Preferably, the locations are selected to exclude infracted locations. Preferably, the selected chamber is the left ventricle and identifying the last activated site preferably comprises determining conduction delays from the right ventricle to a plurality of locations within the myocardium of the left ventricle. The conduction delays may be measured by detecting time delays between right ventricular activation events and corresponding left ventricular activation events.

Preferably the right ventricular activation events include paced right ventricular events. Preferably, an atrioventricular delay is set to be sufficiently short to avoid fusion of between intrinsic ventricular events and paced ventricular events. Positioning the pacing electrode may be performed by positioning a tip of a pacing lead at or near the preferred location.

The method may further comprise inputting a map of the heart of the patient and plotting the preferred location for pacing within the map of the heart using a display device.

Preferably, following determination of the preferred pacing location, the preferred location within the selected chamber is marked.

The above and further features, advantages and benefits of the present system will be apparent upon consideration of the present description taken in conjunction with the accompanying drawings, in which:
FIG. 1 is a flow chart providing an overview of an exemplary technique provided in accordance with illustrative embodiments for identifying optimal locations for positioning the pacing electrodes based on myocardial activation times;
FIG. 2 illustrates an exemplary implantable master/satellite cardiac stimulation system having a master pacing device for delivering stimulation therapy to the right atria and the right ventricle via endocardial electrodes and a satellite pacing device for delivering stimulation therapy to the left ventricle via an epicardial electrode;
FIG. 3 is a flow chart illustrating an exemplary electrical mapping technique for identifying the optimal location for positioning the epicardial electrode of FIG. 2 based on atrial to LV conduction delays;
FIG. 4 illustrates an exemplary implantable cardiac stimulation system having a biventricular stimulation device for delivering therapy to all four chambers of the heart via various endocardial electrodes including an LV electrode;
FIG. 5 is a flow chart illustrating an exemplary electrical mapping technique for identifying the optimal location for positioning the LV electrode of FIG. 4 based on atrial to LV conduction delays;
FIG. 6 is a flow chart illustrating an exemplary mechanical mapping technique for identifying the optimal location for positioning an LV electrode based on atrial to LV conduction delays;
FIG. 7 is a flow chart illustrating an exemplary mapping technique for identifying the optimal location for positioning an LV electrode based on RV to LV conduction delays;
FIG. 8 is a flow chart illustrating an exemplary mapping technique for identifying the optimal location for positioning an LV electrode based on LV to RV conduction delays;
FIG. 9 is a flow chart providing an overview of an exemplary technique for identifying optimal locations for positioning pacing electrodes based on both myocardial activation delays and capture thresholds;
FIG. 10 is a functional block diagram of components of an external programmer device for use with the techniques of FIGS. 1 - 9; and
FIG. 11 is a functional block diagram of internal components of the implantable biventricular stimulation device of FIG. 4.

### Overview of Technique for Identifying Optimal Pacing Location

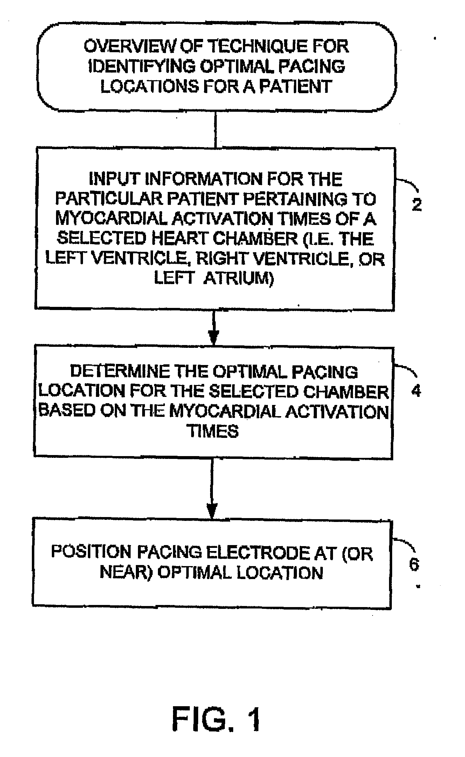

FIG. 1 provides an overview of the technique for identifying optimal or preferred pacing locations within the heart of a particular patient. Briefly, at step 2, information is input for the particular patient pertaining to be myocardial activation times within a cardiac chamber where a pacing electrode is to be implanted (typically the left ventricle or the right ventricle.) Then, at step 4, the optimal pacing location for that chamber is determined based upon the myocardial activation times. Preferably, the location that is the last electrically activated site within a given chamber is identified as being the optimal or preferred pacing location. For example, the last site within the left ventricular myocardium to depolarize during a ventricular contraction represents the optimal pacing location within the left ventricle. Thereafter, at step 6, a pacing electrode is implanted at or near the optimal location for use in delivering pacing therapy. The technique is perhaps most advantageously applied to identify optimal pacing locations in the ventricles for use in delivering CRT but is applicable to other circumstances as well Examples are described below for both master/satellite pacing systems as well as biventricular single device systems.

As noted above in the Summary, the last activated site within the myocardium of a given chamber is believed to be the optimal site for pacing that chamber because it corresponds to portions of the myocardium that would otherwise contract last in response to intrinsic pacing pulses and hence which most significantly contribute to uneven contraction of the chamber. By delivering pacing pulses directly at that location, adjacent portions of the myocardium can be caused to contract sooner thus improving the uniformity of chamber contraction and thereby improving stroke volume from that chamber and hence improving overall cardiac function. The optimal location detection technique is particularly effective for use in identifying pacing sites in the left or right ventricles but is also applicable to identifying pacing sites in the atria as well.

### Overview of Master/Satellite Pacing System

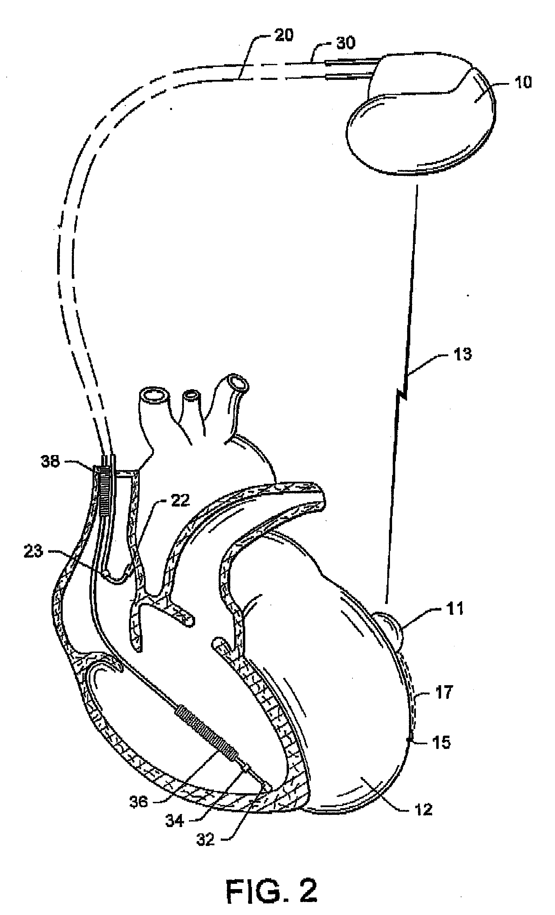

FIG. 2 illustrates a master pacing device 10 in electrical communication with a heart 12 by way of three leads 20 and 30 suitable for delivering multi-chamber stimulation and shock therapy. To sense atrial cardiac signals and to provide right atrial chamber stimulation therapy, the master pacing device is coupled to an implantable right atrial lead 20 having at least an atrial tip electrode 22, which typically is implanted in the right atrial appendage. The master pacing device is also shown in electrical communication with the heart by way of an implantable right ventricular lead 30 having, in this embodiment, a right ventricular tip electrode 32, a right ventricular ring electrode 34, a right ventricular (RV) coil electrode 36, and a superior vena cava (SVC) coil electrode 38. Typically, the right ventricular lead 30 is transvenously inserted into the heart so as to place right ventricular tip electrode 32 in the right ventricular apex so that RV coil electrode 36 will be positioned in the right ventricle and SVC coil electrode 38 will be positioned in the superior vena cava. Accordingly, right ventricular lead 30 is capable of receiving cardiac signals and delivering stimulation in the form of pacing and shock therapy to the right ventricle.

FIG. 2 also illustrates a satellite pacing device 11. The satellite pacing device is mounted on the left side of the heart, particularly to the epicardium of the left ventricle. The satellite pacing device can be mounted, for example, using a thoracoscopic procedure during implant of the master pacing device. The satellite pacing device communicates with the master pacing device using wireless communication technologies, such as high frequency modulation, as represented by transmission link 13. The satellite pacing device delivers pacing pulses to the epicardium of the left ventricular via an electrode 15 coupled to the satellite device via lead 17.

The master/satellite system is especially advantageous for use in performing CRT wherein pacing pulses with carefully chosen time delays are delivered to the right ventricle via endocardial lead 30 under control of the master device 10 and to the left ventricle via epicardial electrode 15 under control of the satellite device 11. An example of a master/satellite system, which may be adapted for use in performing CRT, is described in U.S. Patent Application 10/408,198, entitled "Implantable Cardiac System with Master Pacing Unit and Slave Pacing Unit", filed April 3, 2003, and is fully incorporated by reference herein.

### Exemplary Electrical Mapping Technique for Identifying Optimal LV Epicardial Pacing Location Based on Time Delays from Atria

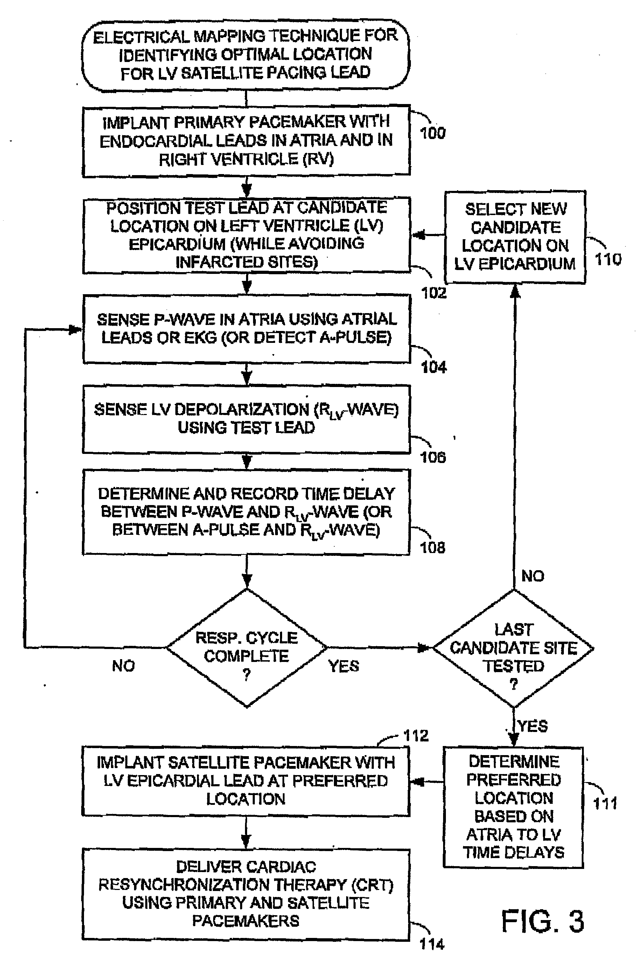

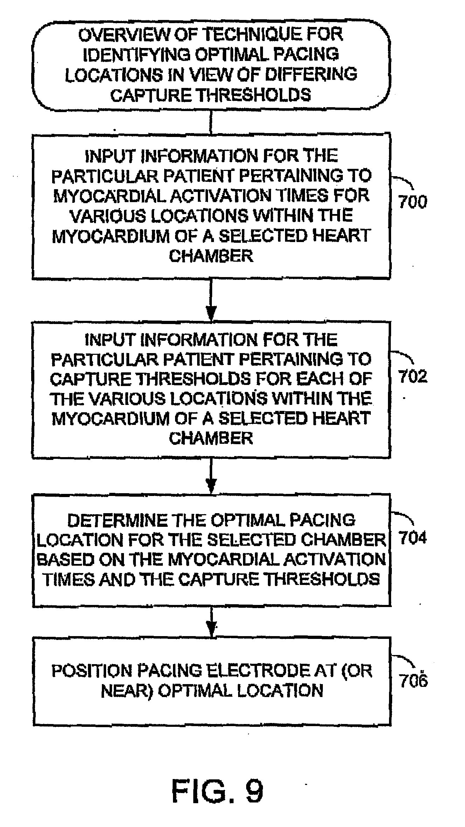

FIG. 3 illustrates an exemplary electrical mapping technique for identifying the optimal location for positioning pacing electrode 15 of left ventricular satellite pacer 11 of FIG. 2 based on atrial to left ventricular propagation times delays. Initially, at step 100, the primary pacemaker is implanted within the patient and leads are implanted in the right atrium and the right ventricle, i.e. leads 20 and 30 of FIG. 2 are implanted. Then, at step 102, a test electrode is positioned at a candidate location on the left ventricular epicardium, such as somewhere in the lateral, anterior, posterior and apical regions of the let ventricle. Note that infarcted sites are avoided since such sites are not likely to respond to pacing stimulation. Infarcted sites may be identified using otherwise conventional techniques. (An exemplary technique for identifying infarcted sites based on an analysis of the morphology of evoked response (ER) is discussed below in connection with FIG. 9.) The test electrode may be electrode 15 of the satellite pacer of FIG. 2 or an electrode within a separate sensing probe. For example, the sensing probe may be: a stand alone mapping catheter having either a unipolar or a bipolar lead; a lead introducer with either a unipolar or bipolar lead at its distal end; or an epicardial lead prior to lead fixation. If a mapping catheter is employed as the test electrode, any of a variety of designs can be used. U.S. Patent 4,402,323 to *White,* entitled "Disposable Electrophysiological Exploring Electrode Needle", describes an epicardial lead with disposable mapping attachment at its distal end. In any case, at step 104, an atrial electrical event is sensed either by a surface ECG or by the master device using tip and ring electrodes implanted within the right atrium. The atrial event may be either an intrinsic depolarization (i.e. P-wave) or a paced event (i.e. an A-pulse). At step 106, the resulting ventricular depolarization (i.e. R_{LV}-wave) is sensed in the left ventricular epicardium using the test electrode.

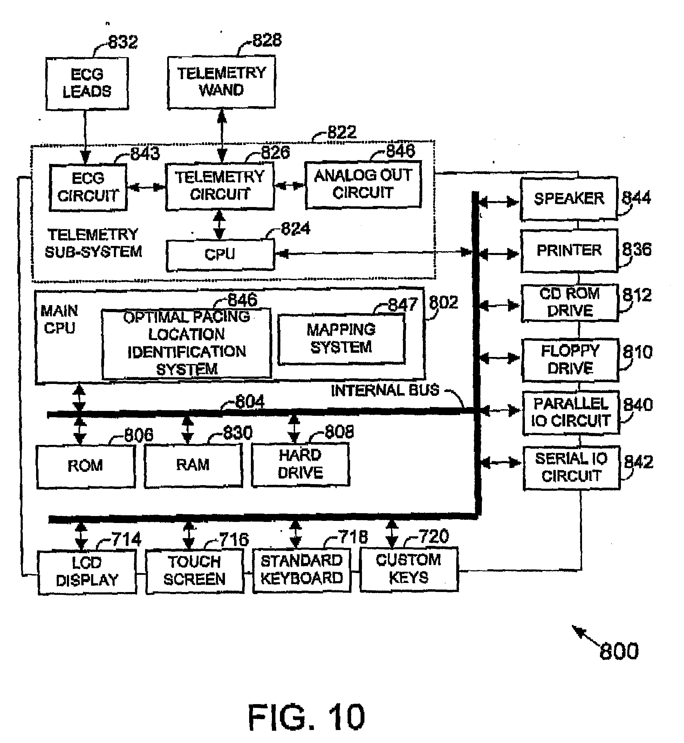

At step 108, the time delay between atrial activation and the resulting LV activation is detected and recorded. In other words, the time delay from detection of the peak of the P-wave (or A-pulse) within the right atrium and the detection of the peak of the resulting R_{LV}-wave within the left ventricle is calculated (i.e. PR_{LV} or AR_{LV}). This represents the conduction time between the right atrium and the candidate location. This calculation is preferably performed by an external programmer device (shown in FIG. 10) based on data transmitted thereto. Preferably, conduction delays for the number of beats corresponding to at least one respiration cycle are detected and averaged within steps 104 - 108. In other words, if there are twenty heartbeats in one respiration cycle, steps 104 - 108 are repeated twenty times. The number of beats needed to cover one respiration cycle may be calculated based on heart rate and respiration rate.

The test electrode is then moved to a new location at step 110, another atrial event is detected, and the propagation time delay between the right atrium and the new location on the left ventricular epicardium is detected and stored. This process is repeated dozens of times while moving the test electrode around the left ventricular epicardium until all of the candidate locations have been tested. Then, at step 111, the preferred or optimal location is determined based on the various right atria (RA) to LV time delays. This may be performed simply be selecting the location having the longest time based on data recorded at step 108. In a technique describe below in connection with FIG. 9, capture thresholds may also be taken into account to ensure that a location is not chosen that will require to high of a pacing pulse magnitude.

Once the optimal or preferred location is identified, the satellite pacemaker is then implanted, at step 112, with its epicardial electrode positioned at the location identified as providing the longest time delay. If desired, prior to the actual mounting of the epicardial pacing electrode, the effectiveness of the optimal location may be verified by applying pacing pulses using the electrode while measuring cardiac function. Then, beginning at step 114, the master pacemaker and the satellite pacemaker are controlled to coordinate the delivery of CRT (or other appropriate therapy) by selectively applying ventricular pacing pulses to the right and left ventricles using the right ventricular lead of the master pacemaker and the epicardial lead of the satellite pacemaker (as well as the atrial leads of the master pacemaker.)

Note that, once the longest time delay is found at step 111, the location may then be marked with a contrast agent so that the location can be easily identified later for mounting the epicardial electrode. Contrast agents for use in marking myocardial tissue are well known in the art. Alternatively, the best location within each of a set of various regions of the left ventricular can be separately marked for later selection. In still other implementations, the locations are not marked. Instead, when it comes time to implant the satellite pacemaker, the satellite pacemaker is mounted within a region know to contain the best pacing locations and then the specific location for mounting pacing electrode 15 is re-identified within that region by again performing steps 102 - 111.

As noted, the actual determination of the optimal location performed at step 111 is preferably performed by an external programmer (or other external device). Otherwise conventional software programming techniques may be used to develop software for averaging the delay times recorded at each candidate location, for determining the optimal location from the averaged values, and for displaying the results on the external programmer for view by the physician or other medical personnel. Graphics software may be employed for displaying a digital map or model of the heart of the patient along with an indication of the optimal pacing location so that the location can be easily visualized. In some cases, the physician may choose to mount the epicardial electrode at a location that is not necessarily optimal in terms of delay times but that has other countervailing advantages. Accordingly, the relative conduction delay times of the other locations tested are also preferably displayed for comparison purposes. The map or model of the heart is preferably a 3-D representation, which may be based on the actual heart of the patient and generated based upon CAT scans or other 3-D imaging techniques. Exemplary 3-D cardiac imaging techniques are set forth in U.S. Patent 5,687,737 to *Branham et al*., entitled "Computerized Three-Dimensional Cardiac Mapping with Interactive Visual Displays." See also U.S. Patent 6,625,482 to *Panescu et al*., entitled "Graphical User Interface for Use with Multiple Electrode Catheters." The aforementioned patent application to *Ben-Haim* also discusses techniques for generating maps of the heart. Alternatively, the 3-D representation may be a generic representation of a human heart. In either case, otherwise conventional techniques may be employed for digitizing the various locations of the test electrode so that the locations can be displayed in conjunction with the representation of the heart. Exemplary techniques for computing electrode locations in 3-D coordinates are discussed in U.S. Patent 5,485,849 to *Panescu et al*. See also U.S. Patent 6,246,898 to Vesely *et al*., entitled "Method for Carrying out a Medical Procedure Using a Three-Dimensional Tracking and Imaging System." An added advantage of recording a 3-D representation of the heart along with the conduction times at various locations on the epicardium is that, if the electrode needs to be repositioned later, the entire process need not be repeated. Rather the physician can instead just review the delay times as displayed in conjunction with the model of the heart and select an alternate epicardial pacing location.

Although described with reference to an LV epicardial electrode, the techniques of FIG. 3 are equally applicable to identifying the optimal location for an RV satellite pacer epicardial electrode. Indeed, optimal locations for both LV and RV epicardial pacing may be identified. In one example, the optimal location for the RV epicardium is first determined based on propagation time delays from the atria to the right ventricle then the optimal time delay for the LV epicardium is determined based on propagation time delays from the atria to the left ventricle. Moreover, the techniques may be adapted for identifying optimal locations for atrial epicardial pacing, if such is desired. For example, time delays may be detected between right atrial pacing events (such as A-pulses generated by RA lead 220) and resulting left atrial depolarizations (P_{LV}-wave) sensed via a sensing probe positioned on the left atrium epicardium. The time delays are then used to identify the optimal location for a left atrial (LA) epicardial pacing lead. The technique can also be extended to identifying an optimal location for right atrial (RA) epicardial pacing, preferably for use in combination with an endocardial RA pacing lead.

Thus far, techniques for identifying an optimal pacing location for epicardial electrodes have been described. In the next sections, techniques for identifying the optimal location for positioning tip electrodes of an internal pacing leads will be described.

### Overview of the Biventricular Single Device Stimulation System

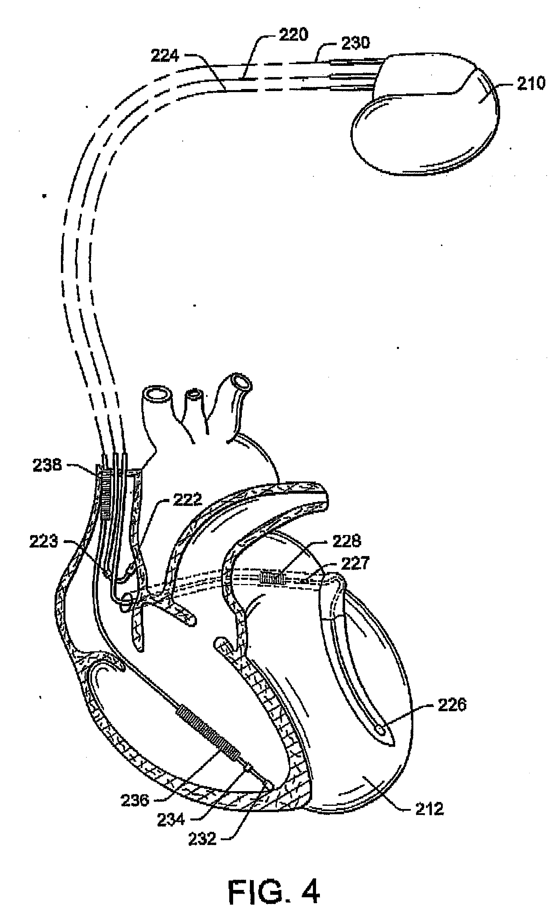

In FIG. 4, a simplified block diagram is shown of a dual-chamber implantable stimulation device 210, which is capable of treating both fast and slow arrhythmias with stimulation therapy, including cardioversion, defibrillation, and pacing stimulation. To provide atrial chamber pacing stimulation and sensing, stimulation device 210 is shown in electrical communication with a heart 212 by way of a left atrial lead 220 having an atrial tip electrode 222 and an atrial ring electrode 223 implanted in the atrial appendage. The stimulation device 210 is also in electrical communication with the heart by way of a right ventricular lead 230 having, in this embodiment, a ventricular tip electrode 232, a right ventricular ring electrode 234, a right ventricular coil electrode 236, and a SVC coil electrode 238. Typically, the right ventricular lead 230 is transvenously inserted into the heart so as to place the RV coil electrode 236 in the right ventricular apex, and the SVC coil electrode 238 in the superior vena cava. Accordingly, the right ventricular lead is capable of receiving cardiac signals, and delivering stimulation in the form of pacing and shock therapy to the right ventricle.

To sense left atrial and ventricular cardiac signals and to provide left chamber pacing therapy, the stimulation device 210 is coupled to a "coronary sinus" lead 224 designed for placement in the "coronary sinus region" via the coronary sinus os for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus. Accordingly, an exemplary coronary sinus lead 224 is designed to receive atrial and ventricular cardiac signals and to deliver left ventricular pacing therapy using at least a left ventricular tip electrode 226, left atrial pacing therapy using at least a left atrial ring electrode 227, and shocking therapy using at least a left atrial coil electrode 228. With this configuration, biventricular pacing can be performed. Although only three leads are shown in FIG. 4, it should also be understood that additional stimulation leads (with one or more pacing, sensing and/or shocking electrodes) may be used in order to efficiently and effectively provide pacing stimulation to the left side of the heart or atrial cardioversion and/or defibrillation.

### Exemplary Electrical Mapping Technique for Identifying Optimal LV Endocardial Pacing Location Based on Time Delays from Atria

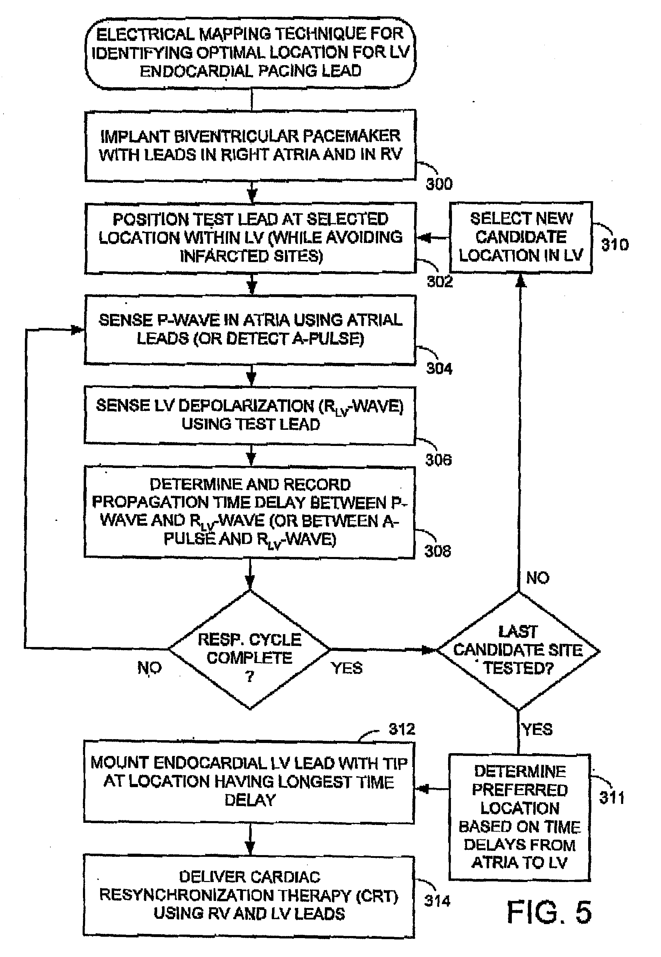

FIG. 5 illustrates an exemplary electrical mapping technique for identifying the optimal location for mounting tip 226 of left the ventricular lead 224 of FIG. 2 based on atrial to ventricular delays. Many of the steps of FIG. 5 are similar to those of FIG. 3 and only pertinent differences will be described in detail. Beginning at step 300, a biventricular pacemaker is implanted within the patient with leads 220 and 230 mounted within the atria and in the right ventricle. Beginning at step 302, a sensing probe (or the tip 226 of LV lead 224) is positioned at a candidate location against the left ventricular endocardium. Note that, as before, infarcted sites are avoided. An exemplary endocardial sensing probe for use within the left ventricle is set forth in U.S. Patent 4,777,955 to *Brayton et al*., entitled "Left Ventricle Mapping Probe." At step 304, an atrial event is sensed by the pacemaker using atrial tip and ring electrodes 222 and 223. As before, the atrial event may be either an intrinsic depolarization (i.e. P-wave) or a paced event (i.e. an A-pulse). At step 306, the resulting LV depolarization (R_{LV}-wave) is sensed using the LV lead. The propagation time delay between the atrial event and the LV depolarization is determined at step 308 and this value is recorded, preferably using an external device (FIG. 10). Accordingly, the atrial and ventricular data sensed at steps 304 and 306 is first transmitted to the external programmer. In addition, as in the preceding embodiment, data for the number of beats corresponding to at least one respiration cycle is preferably recorded for the candidate location. Then, a new endocardial location is selected at step 310 and the process is repeated. Once all of the candidate locations have been tested then, at step 311, the preferred or optimal location is determined based on the various RA to LV time delays. A contrast agent may be used to mark the optimal location for subsequent mounting of the tip of lead 230. An endocardial sensing lead with capability for delivering a contrast agent is set forth in U.S. Patent 6,377,856 to *Carson,* entitled "Device and Method for Implanting Medical Leads." At step 312, the tip of a left ventricular pacing lead is mounted at or near the optimal location. Beginning at step 314, CRT (or other appropriate pacing therapy) is delivered the using the pacemaker via the left and right ventricular leads and the atrial leads.

As with the technique of FIG. 3, any appropriate imaging techniques may be used to display the optimal pacing location in conjunction with a map or model of the heart. To this end, a suitable 3-D location digitizing technique can be used while the tip of the lead is placed at various locations within the heart to allow the location of the tip of the lead to be recorded along with the corresponding time delay value for display along with a 3-D map of the heart (generic or otherwise). A technique for mapping the interior of the left ventricle is discussed in Bøtker *et al*., "Electromechanical Mapping for Detection of Myocardial Viability in Patients With Ischemic Cardiomyopathy", Circulation 2001;103:1631-1637.

Although described with reference to an LV lead, the technique of FIG. 5 is equally applicable to identifying the optimal location for tip 232 of RV lead 230 and to identifying optimal locations for both LV and RV endocardial pacing. For example, the optimal location for the RV endocardium is first determined based on propagation time delays from the atria to the right ventricle then the optimal time delay for the LV endocardium is determined based on propagation time delays from the atria to the left ventricle. In addition, the techniques may be adapted for identifying optimal locations for endocardial left atrial pacing. In other words, the technique may be used to identify a location for mounting an endocardial left atrial pacing lead (not specifically shown in FIG. 4.) Time delays may be detected between right atrial pacing events (such as A-pulses generated by RA lead 220) and resulting left atrial depolarizations (P_{LV}-wave) sensed via a sensing probe positioned within the left atrium. The time delays are used to identify the optimal location for an RA endocardial pacing lead. The last electrically activated site within the RA endocardium is identified as the preferred location.

In the following, various alternative mapping techniques will be described including techniques exploiting mechanical, rather than electrical, myocardial activation times and techniques that exploit LV to RV delays or RV to LV delays, rather than atrial to ventricular delays. The devices and systems illustrated in FIGS. 2 and 4 may be utilized in connection with the implementing the techniques of the remaining figures as well.

### Exemplary Mechanical Mapping Technique for Identifying Optimal LV Epicardial Pacing Location Based on Time Delay from Atria

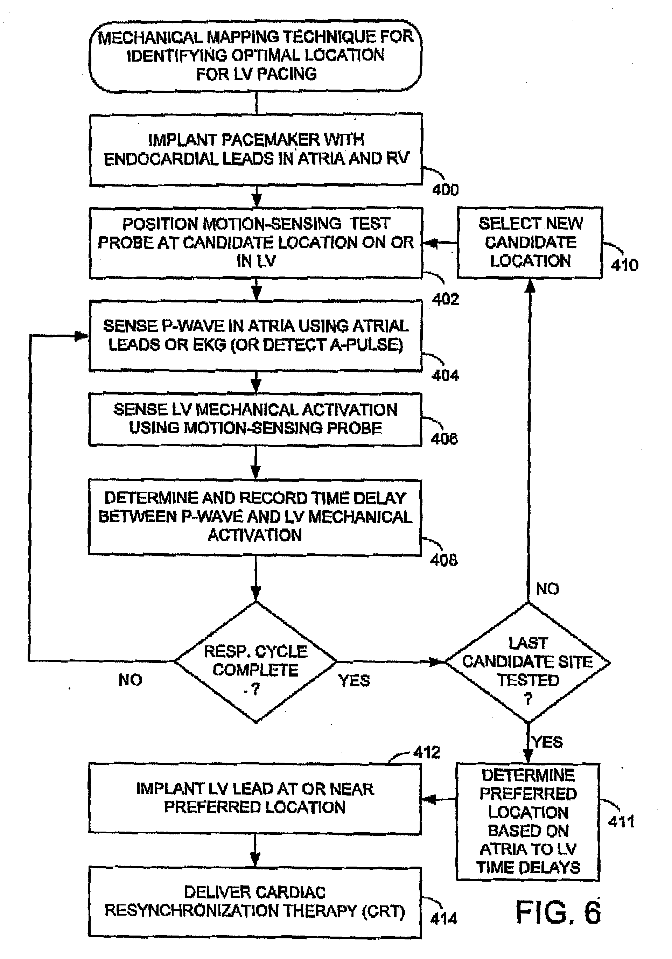

FIG. 6 illustrates an exemplary mechanical mapping technique for identifying the optimal location for positioning a pacing electrode based on physical contraction of the myocardium of the heart. Many of the steps of FIG. 6 are similar to those of FIGS. 3 and 5 and only pertinent differences will be described in detail. Beginning at step 400, a master pacer or biventricular pacemaker is implanted within the patient with leads 220 and 230 mounted within the atria and in the right ventricle. Beginning at step 402, a motion-sensing probe is positioned at a candidate location in or on the left ventricular myocardium. The motion-sensing probe may be any probe capable of sensing the physical (or mechanical) contraction of portions of the myocardium of the heart. This is in contrast to an electrical sensing probe for sensing the electrical depolarization of the myocardium. The motion-sensing probe may incorporate both electrical and mechanical components and may, for example, contain an accelerometer or similar motion sensor. Medical probes employing motion-sensing accelerometers are discussed in U.S. Patent 5,552,645 to Weng. Alternatively, an electromagnetic 3D sensor may be employed or a sensor designed to detect longitudinal contraction of myocytes. In any case, if the technique is being performed to identify an epicardial pacing location, then the motion-sensing probe is preferably placed adjacent the LV epicardium. If the technique is being performed to identify an endocardial pacing location, then the motion-sensing probe is preferably placed adjacent an inner wall of the LV. As before, infarcted sites are avoided.

At step 404, an atrial event is sensed by the pacemaker using atrial tip and ring electrodes. As before, the atrial event may be either an intrinsic depolarization (i.e. P-wave) or a paced event (i.e. an A-pulse). At step 406, the resulting contraction of the portion of the myocardium adjacent the probe during LV depolarization is sensed using the motion-sensing probe. The propagation time delay between the atrial event and the mechanical contraction of the adjacent portion of the LV myocardium is determined at step 408 and this value is recorded, preferably using the external device of FIG. 10. Data for the number of beats corresponding to at least one respiration cycle is preferably recorded for the candidate location. Then, a new candidate location is selected at step 410 and the process is repeated. Once all of the candidate locations have been tested then, at step 411, the preferred or optimal location is determined based on the various RA to LV time delays. As before, a contrast agent may be used to mark the optimal location for subsequent mounting of LV electrode. At step 412, the LV pacing electrode is then mounted at or near the optimal location (epicardial or endocardially, as needed). Beginning at step 414, CRT (or other appropriate therapy) is then delivered.

As with the aforementioned techniques, any appropriate imaging techniques may be used to display the optimal pacing location in conjunction with a map or model of the heart. Moreover, although described with reference to an LV lead, the technique of FIG. 6 is equally applicable to identifying the optimal location for an RV electrode or to identifying optimal locations for both LV and RV pacing. In addition, the techniques may be adapted for identifying optimal locations for left atrial pacing. Also, rather than using a motion-sensing probe, the technique of FIG. 6 may instead exploit tissue Doppler imaging techniques wherein the location within a given chamber having the last activation time is identified by examining contraction of various portions of the myocardium of the chamber via Doppler imaging. An example of a Doppler imaging technique is set forth in U.S. Patent 6,650,927 to Keidar, entitled "Rendering of Diagnostic Imaging Data on a Three-Dimensional Map", which his incorporated by reference herein. Also, techniques set forth in the patents referenced above to Branham et al., Panescu et al., Ben-Haim and Vesely et al. may be exploited in connection with detecting and/or mapping the physical contraction of portions of the myocardium of the heart. See also U.S. Patent 6,484,118, entitled "Electromagnetic Position Single Axis System" to Govari; U.S. Patent 6,456,867, entitled "Three-Dimensional Reconstruction of Intrabody Organs" to Reisfeld; 6,301,496, entitled "Vector Mapping of Three-Dimensionally Reconstructed Intrabody Organs and Method of Display" also to Reisfeld; and U.S. Patent 6,226,542, entitled "Three-Dimensional Reconstruction Of Intrabody Organs" also to *Reisfeld*.

### Exemplary Mapping Technique for Identifying Optimal LV Epicardial Pacing Location Based on Time Delay from RV to LV

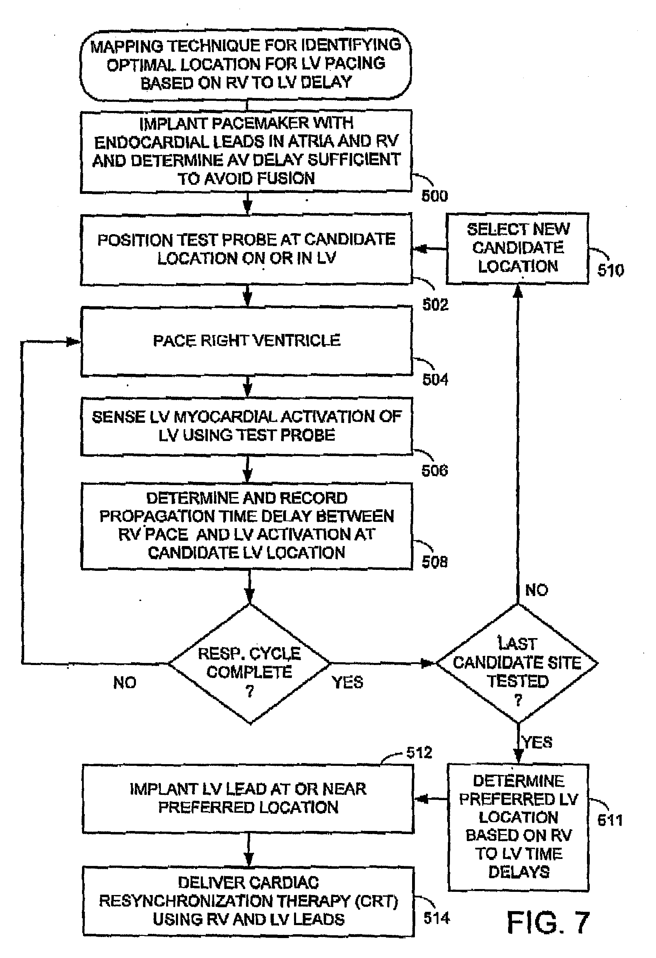

FIG. 7 illustrates an exemplary mapping technique for identifying the optimal location for positing epicardial or endocardial LV pacing leads based on RV to LV time delays. This is in contrast with the foregoing technique that operates based on atrial to ventricular delays. Nevertheless, many of the steps of FIG. 7 are similar to those described above and only pertinent differences will be described in detail. Beginning at step 500, a master pacer or biventricular pacemaker is implanted within the patient with leads 220 and 230 mounted within the atria and in the right ventricle. The lead is the RV is typically mounted in the endocardial apex. In addition, at step 500, an atrioventricular (AV) delay is set to a sufficiently short value to avoid any substantial risk of fusion during RV pacing. In this regard, if the AV delay is to long, then AV conduction from the atria to the ventricles may result in intrinsic depolarization of the ventricles occurring slightly before or contemporaneous with depolarization caused by RV pacing pulses. Techniques for identifying AV delay values sufficient to avoid fusion are discussed in U.S. Patent No. 5,334,220 to *Sholder,* entitled "Dual-Chamber Implantable Pacemaker Having An Adaptive Av Interval That Prevents Ventricular Fusion Beats And Method Of Operating Same", which is incorporated by reference herein.

Beginning at step 502, a motion-sensing or electrical sensing probe is positioned at a candidate location in or on the left ventricular myocardium. If the technique is performed to identify an epicardial pacing location, the probe is preferably placed adjacent the LV epicardium. If the technique is performed to identify an endocardial pacing location, the probe is preferably placed adjacent an inner wall of the LV. As before, infarcted sites are avoided. At step 504, a pacing pulse is delivered to the RV using the RV tip and ring electrodes subject to the aforementioned AV delay. At step 506, the resulting LV depolarization is sensed using either the sensing probe. The propagation time delay between the RV pace and the resulting LV activation is determined at step 508 and this value is recorded using the external device of FIG. 10. Data for the number of beats corresponding to at least one respiration cycle is preferably recorded for the candidate location. A new candidate location is selected at step 510 and the process is repeated. Once all of the candidate locations have been tested then the preferred or optimal location is determined, at step 511, based on the various RV to LV time delays. As before, a contrast agent may be used to mark the optimal location for subsequent mounting of LV electrode. At step 512, the LV pacing electrode is then mounted at or near the optimal location (epicardial or endocardially, as needed). Beginning at step 514, CRT (or other appropriate therapy) is then delivered.

As with the aforementioned techniques, any appropriate imaging technique may be used to display the optimal pacing location in conjunction with a map or model of the heart. Moreover, although described with reference to identifying the optimal location for an LV lead based on RV to LV delays, the technique of FIG. 7 is equally applicable to identifying the optimal location for an RV electrode based on LV to RV delays arising from a fixed LV pacing electrode. Also, rather than using an electrical or a motion-sensing sensing probe, the technique of FIG. 7 may instead exploit tissue Doppler imaging techniques to detect mechanical activation time delays.

### Exemplary Mapping Technique for Identifying Optimal LV Epicardial Pacing Location Based on Time Delay from LV to RV

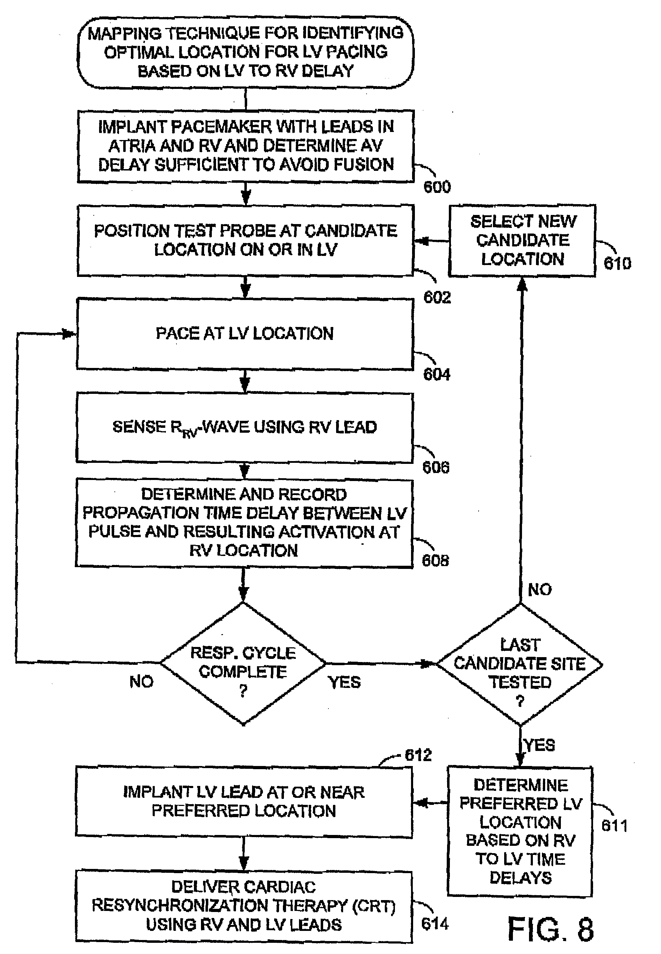

FIG. 8 illustrates an exemplary mapping technique for identifying the optimal location for positing epicardial or endocardial LV pacing leads based on LV to RV time delays triggered by LV pacing. In other words, with this technique, a pacing probe is used to apply pacing pulses to the LV. This is in contrast with the foregoing techniques that operate using sensing probes for sensing LV activation. Nevertheless, many of the steps of FIG. 8 are similar to those described above and only pertinent differences will be described in detail. Beginning at step 600, a master pacer or biventricular pacemaker is implanted within the patient with leads 220 and 230 mounted within the atria and in the right ventricle. In addition, at step 600, the AV delay is set to a sufficiently short value to avoid any substantial risk of fusion during LV pacing. Beginning at step 602, an electrical pacing probe is positioned at a candidate location in or on the left ventricular myocardium. A suitable pacing/mapping probe is set forth in the above-referenced patent to *White.* If the technique is performed to identify an epicardial pacing location, the pacing probe is preferably placed adjacent the LV epicardium. If the technique is performed to identify an endocardial pacing location, the pacing probe is preferably placed adjacent an inner wall of the LV. As before, infarcted sites are avoided.

At step 604, an LV pacing pulse is delivered by an external device using the LV pacing probe based on the AV delay. At step 606, a resulting RV depolarization is sensed using the electrodes mounted in the right ventricle (i.e. electrodes 32 and 34 of FIG. 2 or electrodes 232 and 234 of FIG. 4, depending upon the implementation.) The propagation time delay between the LV pacing pulse and the resulting RV activation is determined at step 608 and this value is recorded using the external device of FIG. 10. Data for the number of beats corresponding to at least one respiration cycle is preferably recorded for the candidate location. A new candidate location is then selected at step 610 and the process is repeated. Once all of the candidate locations have been tested then the preferred or optimal location is determined, at step 611, based on the various LV to RV time delays. As before, a contrast agent may be used to mark the optimal location for subsequent mounting of an LV electrode. At step 612, an LV pacing electrode is then permanently mounted at or near the optimal location (epicardial or endocardially, as needed). If the LV pacing probe is intended to be used as the permanent LV pacing electrode then it may be mounted at the optimal location immediately. In any case, beginning at step 614, CRT or other appropriate therapy is then delivered.

As with the aforementioned techniques, any appropriate imaging techniques may be used to display the optimal pacing location in conjunction with a map or model of the heart. Moreover, although described with reference to identifying the optimal location for an LV lead based on LV to RV delays, the technique of FIG. 8 is equally applicable to identifying the optimal location for an RV electrode based on RV to LV delays triggered by an RV pacing probe and detected by a fixed LV electrode.

### Overview of Technique for Identifying Optimal Pacing Location while Taking into Account Capture Thresholds

FIG. 9 provides an overview of a technique for identifying optimal pacing locations for a particular patient while taking into account capture thresholds. More specifically, the technique of FIG. 9 operates to determine an optimal pacing location based on both the activation time delay and on the capture threshold so as to ensure that a pacing location is not selected that has a capture threshold that is too high and in particular to exclude sites that cannot be electrically activated by a pacing electrode, perhaps because the myocardial tissue at that site was subject to infarction. The technique may be used in conjunction with any of the exemplary techniques described above.

Briefly, at step 700, information is input for the particular patient pertaining to be myocardial activation times for various locations on or within a cardiac chamber where a pacing electrode is to be implanted (such as within the left ventricle). This is performed in accordance with the techniques described above. Additionally, at step 702, information is input pertaining to be capture thresholds for each of the various locations of step 700. This information may be generated using a pacing probe. Then, at step 704, the optimal pacing location is determined based upon both the myocardial activation times and the capture thresholds. In one example, a maximum acceptable capture threshold is specified and then the location having the latest activation time is selected only from among locations with capture thresholds not exceeding the threshold. Thereafter, at step 706, a pacing electrode is implanted at or near the optimal location for use in delivering pacing therapy.

The capture-based technique of FIG. 9 is perhaps most advantageously applied for use in connection with the mapping technique of FIG. 8, which employs a pacing probe that may also be used to identify pacing sites having acceptable capture thresholds. In one example, at step 604, the pacing probe is controlled to deliver pacing pulses at a candidate site and capture is verified. If capture cannot be verified, then the site is excluded. If capture is verified, then the activation time for that site is measured for use in determining the optimal pacing location. Another technique for determining whether to exclude sites based on capture threshold is to examine the morphology of the evoked response (ER) -- assuming a response is evoked. The pertinent parameters of the ER morphology include pacing latency (i.e. the time delay from the pacing pulse to a minimum ER or to the onset of ER or to the maximum slope (DMAX)), the area integral below the rest potential or paced depolarization integral (PDI) and the value of DMAX). Assuming a constant pacing pulse amplitude, a site is excluded if the value of the morphological parameter is below or above a predetermined threshold, which may be set based on routine experimentation. The ER-based technique can thus be used to identify infarcted or ischemic sites.

However, the capture-based technique may also be employed in connection with the other mapping techniques described herein. For example, mapping techniques may be performed by first using a sensing probe to identify one optimal location within each region of the heart chamber being tested -- based solely on activation times. Thereafter, a pacing probe is placed at each of the identified locations to determine whether its capture threshold is acceptable, then the physician selects a particular location having an acceptable capture threshold for actually implanting a pacing electrode. As can be appreciated, the capture thresholds-based techniques of FIG. 9 may be exploited in a wide variety of specific implementations.

### Overview of Exemplary External Programmer

FIG. 10 illustrates pertinent components of an external programmer for use in programming an implantable medical device such as a pacemaker or ICD. Briefly, the programmer permits a physician or other user to program the operation of the implanted device and to retrieve and display information received from the implanted device such as IEGM data and device diagnostic data. Additionally, the external programmer receives and displays ECG data from separate external ECG leads that may be attached to the patient. Depending upon the specific programming of the external programmer, programmer 800 may also be capable of processing and analyzing data received from the implanted device and from the ECG leads to, for example, render preliminary diagnosis as to medical conditions of the patient or to the operations of the implanted device. As noted, the programmer is also configured to receive data representative of conduction time delays from the atria to the ventricles and to determine, therefrom, an optimal or preferred location for pacing.

Now, considering the components of programmer 800, operations of the programmer are controlled by a CPU 802, which may be a generally programmable microprocessor or microcontroller or may be a dedicated processing device such as an application specific integrated circuit (ASIC) or the like. Software instructions to be performed by the CPU are accessed via an internal bus 804 from a read only memory (ROM) 806 and random access memory 830. Additional software may be accessed from a hard drive 808, floppy drive 810, and CD ROM drive 812, or other suitable permanent mass storage device. Depending upon the specific implementation, a basic input output system (BIOS) is retrieved from the ROM by CPU at power up. Based upon instructions provided in the BIOS, the CPU "boots up" the overall system in accordance with well-established computer processing techniques.

Once operating, the CPU displays a menu of programming options to the user via an LCD display 814 or other suitable computer display device. To this end, the CPU may, for example, display a menu of specific programming parameters of the implanted device to be programmed or may display a menu of types of diagnostic data to be retrieved and displayed. In response thereto, the physician enters various commands via either a touch screen 816 overlaid on the LCD display or through a standard keyboard 818 supplemented by additional custom keys 820, such as an emergency VVI (EVVI) key. The EVVI key sets the implanted device to a safe VVI mode with high pacing outputs. This ensures life sustaining pacing operation in nearly all situations but by no means is it desirable to leave the implantable device in the EVVI mode at all times.

With regard to the determination of the optimal pacing location, CPU 802 includes an optimal pacing location identification system 846 and a 3-D mapping system 847. The location identification system inputs data representative of time delays from the right atrium to the candidate locations in the ventricles, either from a sensing probe, the implanted device, or an EKG. If received from the implanted device, telemetry wand 828 is used. If received directly from a mapping probe, any appropriate input may be used, such as parallel IO circuit 840 or serial IO circuit 842. Alternatively, although not shown, a separate dedicated input port for receiving signals from a mapping probe may be provided. In any case, as already explained, the external programmer determines the optimal location based on the data. The mapping system displays the optimal location and/or various candidate locations on a map or model of the heart along with the relative propagation time delay values detected at the various location. The map or model is displayed using display 824 based, in part, on 3-D heart model data input from via ports 840 or 842 from, for example, a 3-D imaging system and/or a 3-D location digitizing apparatus capable of digitizing the 3-D location of a sensing probe or other lead. The physician can thereby view the optimal location on the map of the heart to ensure that the location is acceptable before a lead is permanently mounted at that location. In addition, the heart map and all of the time delay data may be recorded for subsequent review, perhaps if the lead needs to be repositioned.

Once all pacing leads are mounted and all pacing devices are implanted (i.e. master pacemaker, satellite pacemaker, biventricular pacemaker), the various devices are programmed. Typically, the physician initially controls the programmer 800 to retrieve data stored within any implanted devices and to also retrieve ECG data from ECG leads, if any, coupled to the patient. To this end, CPU 802 transmits appropriate signals to a telemetry subsystem 822, which provides components for directly interfacing with the implanted devices, and the ECG leads. Telemetry subsystem 822 includes its own separate CPU 824 for coordinating the operations of the telemetry subsystem. Main CPU 802 of programmer communicates with telemetry subsystem CPU 824 via internal bus 804. Telemetry subsystem additionally includes a telemetry circuit 826 connected to telemetry wand 828, which, in turn, receives and transmits signals electromagnetically from a telemetry unit of the implanted device. The telemetry wand is placed over the chest of the patient near the implanted device to permit reliable transmission of data between the telemetry wand and the implanted device.

Typically, at the beginning of the programming session, the external programming device controls the implanted devices via appropriate signals generated by the telemetry wand to output all previously recorded patient and device diagnostic information. Patient diagnostic information includes, for example, recorded IEGM data and statistical patient data such as the percentage of paced versus sensed heartbeats. Device diagnostic data includes, for example, information representative of the operation of the implanted device such as lead impedances, battery voltages, battery recommended replacement time (RRT) information and the like. Data retrieved from the implanted devices is stored by external programmer 800 either within a random access memory (RAM) 830, hard drive 808 or within a floppy diskette placed within floppy drive 810. Additionally, or in the alternative, data may be permanently or semi-permanently stored within a compact disk (CD) or other digital media disk, if the overall system is configured with a drive for recording data onto digital media disks, such as a write once read many (WORM) drive.

Once all patient and device diagnostic data previously stored within the implanted devices is transferred to programmer 800, the implanted devices may be further controlled to transmit additional data in real time as it is detected by the implanted devices, such as additional IEGM data, lead impedance data, and the like. Additionally, or in the alternative, telemetry subsystem 822 receives ECG signals from ECG leads 832 via an ECG processing circuit 834. As with data retrieved from the implanted device itself, signals received from the ECG leads are stored within one or more of the storage devices of the external programmer. Typically, ECG leads output analog electrical signals representative of the ECG. Accordingly, ECG circuit 834 includes analog to digital conversion circuitry for converting the signals to digital data appropriate for further processing within programmer. Depending upon the implementation, the ECG circuit may be configured to convert the analog signals into event record data for ease of processing along with the event record data retrieved from the implanted device. Typically, signals received from the ECG leads are received and processed in real time.

Thus, the programmer receives data both from the implanted devices and from the external ECG leads. Data retrieved from the implanted devices includes parameters representative of the current programming state of the implanted devices. Under the control of the physician, the external programmer displays the current programming parameters and permits the physician to reprogram the parameters. To this end, the physician enters appropriate commands via any of the aforementioned input devices and, under control of CPU 802, the programming commands are converted to specific programming parameters for transmission to the implanted devices via telemetry wand 828 to thereby reprogram the implanted devices. Prior to reprogramming specific parameters, the physician may control the external programmer to display any or all of the data retrieved from the implanted devices or from the ECG leads, including displays of ECGs, IEGMs, and statistical patient information. Any or all of the information displayed by programmer may also be printed using a printer 836.

A wide variety of parameters may be programmed by the physician. In particular, for CRT, the AV delay and the RV-LV delay of the implanted device(s) are set to optimize cardiac function. In one example, the RV-LV delay is first set to zero while the AV delay is adjusted to achieve the best possible cardiac function (measured using any appropriate cardiac function measurement technique). Then, RV-LV delay is adjusted to achieve still further enhancements in cardiac function. With the leads already mounted at optimal locations within the ventricles and with the AV and RV-LV delay values optimized, it is believed that the best possible cardiac function can be achieved for the patient.

Programmer 800 also includes a modem 838 to permit direct transmission of data to other programmers via the public switched telephone network (PSTN) or other interconnection line, such as a T1 line or fiber optic cable. Depending upon the implementation, the modem may be connected directly to internal bus 804 may be connected to the internal bus via either a parallel port 840 or a serial port 842. Other peripheral devices may be connected to the external programmer via parallel port 840 or a serial port 842 as well. Although one of each is shown, a plurality of input output (IO) ports might be provided. A speaker 844 is included for providing audible tones to the user, such as a warning beep in the event improper input is provided by the physician. Telemetry subsystem 822 additionally includes an analog output circuit 846 for controlling the transmission of analog output signals, such as IEGM signals output to an ECG machine or chart recorder.

With the programmer configured as shown, a physician or other user operating the external programmer is capable of retrieving, processing and displaying a wide range of information received from the ECG leads or from the implanted devices and to reprogram the implanted devices if needed. The descriptions provided herein with respect to FIG. 10 are intended merely to provide an overview of the operation of programmer and are not intended to describe in detail every feature of the hardware and software of the device and is not intended to provide an exhaustive list of the functions performed by the device.

### Internal Components of Exemplary Biventricular Single

### Device System

For the sake of completeness, internal components of the biventricular device of FIG. 4 will now be summarized. For systems employing separate master and satellite pacemakers (such as shown in FIG. 2), selected components of the biventricular device of FIG. 4 are included either within the master device, the satellite device, or within both.

Internal components of exemplary master and satellite pacing devices are set forth in the patent application referenced above entitled "Implantable Cardiac System with Master Pacing Unit and Satellite Pacing Unit".

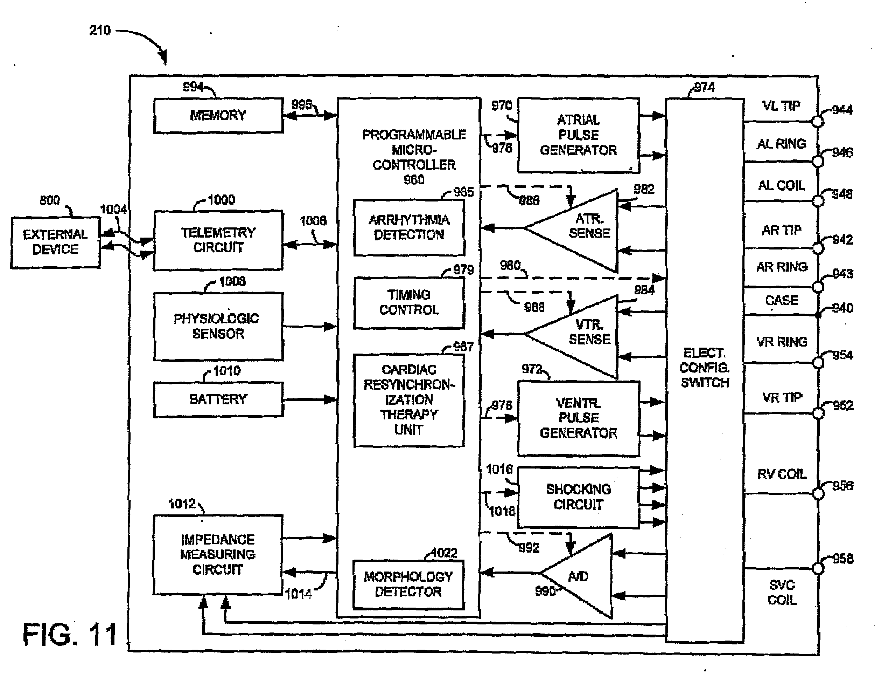

Referring now to FIG. 11, pertinent components of device 210 are described. Housing 940 (shown schematically) for the stimulation device 210 includes a connector (not shown) having an atrial tip terminal 942 adapted for connection to the atrial tip electrode 222 and an atrial ring terminal 943 of the atrial lead 220. The connector further includes a right ventricular tip terminal 952, a ring ventricular ring terminal 954, an RV shocking terminal 956, and an SVC shocking terminal 958 all of which are adapted for connection to the ventricular tip electrode 232, the right ventricular ring electrode 234, the RV coil electrode 236, and the SVC coil electrode 238, respectively. The housing 940 (often referred to as the "can", "case" or "case electrode") acts as the return (common) electrode, or anode, for both the atrial tip electrode 222 and the ventricular tip electrode 232 during unipolar sensing and as the return electrode for just the ventricular tip electrode 232 during Combipolar sensing. Housing 940 can also act as the return (common) electrode, or anode, for the RV coil electrode 236, and the SVC coil electrode 238. For convenience, the names of the electrodes are shown next to the terminals. The left ventricular tip electrode 226, left atrial ring electrode 227, left atrial coil electrode 228, are adapted to be connected to the left ventricular tip terminal 944, left atrial ring terminal 946, and the left atrial coil terminal 948, respectively.

At the core of the stimulation device 210 is a programmable microcontroller 960, which controls the various modes of stimulation therapy. As is well known in the art, the microcontroller 960 includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 960 includes the ability to process or monitor input signals (data) as controlled by a program code stored in a designated block of memory. The details of the design and operation of the microcontroller 960 are not critical. Rather, any suitable microcontroller 960 may be used that carries out the functions described herein. The use of microprocessor-based control circuits for performing timing and data analysis functions is well known in the art.

As shown in FIG. 11, an atrial pulse generator 970 and a ventricular pulse generator 972 generate pacing stimulation pulses for delivery by the atrial lead 220 and the ventricular lead 230, respectively, via a switch bank 974. Ventricular pulse generator is capable of generating separate pulses for delivery to the right and left ventricles in accordance with biventricular pacing techniques. The pulse generators, 970 and 972, are controlled by the microcontroller 960 via appropriate control signals, 976 and 978, respectively, to trigger or inhibit the stimulation pulses. The microcontroller 960 further includes a timing control unit that controls the operation of the stimulation device timing of such stimulation pulses that is known in the art. The microcontroller 960 may also include an AutoCapture threshold detection system, though AutoCapture threshold detection system is not necessary for the purposes of the system and method for determining the optimal LV site. The switch bank 974 includes a plurality of switches for switchably connecting the desired electrodes to the appropriate I/O circuits, thereby providing complete electrode programmability. Accordingly, the switch bank 974, in response to a control signal 980 from the microcontroller 960, sets the polarity of the stimulation pulses by selectively closing the appropriate combination of switches (not shown) as is known in the art.

An atrial sense amplifier 982 and a ventricular sense amplifier 984 are also coupled to the atrial and ventricular leads 220 and 230, respectively, through the switch bank 974 for detecting the presence of cardiac activity.

Sense amplifier 984 is capable of separately sensing signals from both the right and left ventricles in accordance with biventricular pacing techniques. The switch bank 974 determines the "sensing polarity" of the cardiac signal by selectively closing the appropriate switches, as is also known in the art. In this way, the clinician may program the sensing polarity independent of the stimulation polarity. The switch bank also permits the pacemaker to be set to either unipolar sensing or Combipolar sensing. For unipolar sensing, the V TIP and CASE terminals are connected to the ventricular sense amplifier for sensing a voltage differential there between and the A TIP and CASE terminals are connected to the atrial sense amplifier for sensing a voltage differential there between. For Combipolar sensing, the V TIP and CASE terminals are likewise connected to the ventricular sense amplifier but the A TIP and V TIP terminals are connected to the atrial sense amplifier for sensing a voltage differential between the tips of the atrial and ventricular leads.

Each sense amplifier, 982 and 984, preferably employs a low power, precision amplifier with programmable gain and/or automatic gain control, bandpass filtering, and a threshold detection circuit, known in the art, to selectively sense the cardiac signal of interest. The automatic gain control enables the device 210 to deal effectively with the difficult problem of sensing the low frequency, low amplitude signal characteristics of ventricular fibrillation. The gain control is actuated by the programmable micro controller 960. The gains are controlled on the ventricular sense amplifier 984 by the microcontroller using control line 988 and on the atrial sense amplifier 982 on control line 986. The outputs of the atrial and ventricular sense amplifiers, 982 and 984, are connected to the microcontroller 960 which, in turn, inhibits the atrial and ventricular pulse generators, 970 and 972, respectively, in a demand fashion whenever cardiac activity is sensed in the respective chambers.

For arrhythmia detection, the atrial and ventricular sense amplifiers, 982 and 984, sense cardiac signals to determine whether a rhythm is physiologic or pathologic. As used herein "sensing" is reserved for the noting of an electrical depolarization, and "detection" is the processing of these sensed depolarization signals and noting the presence of an arrhythmia. The timing intervals between sensed events (e.g., the P-P and R-R intervals) are then classified by the microcontroller 960 by comparing them to a predefined rate zone limit (i.e., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, activation of special algorithms such as automatic mode switch or high atrial rate episode logging, anti-tachycardia pacing, cardioversion shocks or defibrillation shocks, also known as "tiered therapy"). An arrhythmia detection unit 985 of the microcontroller oversees arrhythmia detection. A cardiac resynchronization therapy unit 987 oversees CRT.

Cardiac signals are also applied to the inputs of an analog to digital (A/D) data acquisition system 990. The gain of the A/D converter 990 is controlled by the microprocessor 960 in order to match the signal amplitude and/or the resolution to a range appropriate for the function of the A/D converter 990. The data acquisition system 990 is configured to acquire intracardiac electrogram signals, convert the raw analog data into a digital signal, and store the digital signals for later processing and/or telemetric transmission to an external device 102. The data acquisition system 990 is coupled to the atrial and ventricular leads, 220 and 230, through the switch bank 974 to sample cardiac signals across any pair of desired electrodes.

The microcontroller 960 is further coupled to a memory 994 by a suitable data/address bus 996, wherein the programmable operating parameters used by the microcontroller 960 are stored and modified, as required, in order to customize the operation of the stimulation device 210 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the heart 212 within each respective tier of therapy.

Advantageously, the operating parameters of the implantable device 210 may be non-invasively programmed into the memory 994 through a telemetry circuit 1000 in telemetric communication with an external device 1002, such as a programmer, transtelephonic transceiver, or a diagnostic system analyzer. The telemetry circuit 1000 is activated by the microcontroller 960 by a control signal 1006. The telemetry circuit 1000 advantageously allows intracardiac electrograms and status information relating to the operation of the device 210 (as contained in the microcontroller 960 or memory 994) to be sent to the external device 1002 through an established communication link 1004.

In the preferred embodiment, the stimulation device 210 further includes a physiologic sensor 1008. Such sensors are commonly called "rate-responsive" sensors. The physiological sensor 1008 is used to detect the exercise state of the patient, to which the microcontroller 960 responds by adjusting the rate and AV Delay at which the atrial and ventricular pulse generators, 970 and 972, generate stimulation pulses. The type of sensor used is not critical and is shown only for completeness.

The stimulation device additionally includes a battery 1010 that provides operating power to all of the circuits shown in FIG. 11. For the stimulation device 210, which employs shocking therapy, the battery must be capable of operating at low current drains for long periods of time and then be capable of providing high-current pulses (for capacitor charging) when the patient requires a shock pulse (preferably, in excess of 2 A, at voltages above 2 V, for periods of 10 seconds or more). The battery 1010 must also have a predictable discharge characteristic so that elective replacement time can be detected. Accordingly, the system preferably employs lithium/silver vanadium oxide batteries, as is true for most (if not all) such devices to date. As further shown in FIG. 11, the device preferably includes an impedance measuring circuit 1012, which is enabled by the microcontroller 960 by a control signal 1022. The impedance measuring circuit 1012 is not critical and is shown for only completeness.

Depending upon the implementation, the device may function as an implantable cardioverter/defibrillator (ICD) device. That is, if it detects the occurrence of an arrhythmia, it automatically applies an appropriate electrical shock therapy to the heart aimed at terminating the detected arrhythmia. To this end, the microcontroller 960 further controls a shocking circuit 1016 by way of a control signal 1018. The shocking circuit 1016 generates shocking pulses of low (up to 0.5 joules), moderate (0.5 to 10 joules), or high energy (11 to 40 joules), as controlled by the microcontroller 960. Such shocking pulses are applied to the patient's heart through at least two shocking electrodes, as shown in this embodiment, using the RV and SVC coil electrodes, 236 and 238, respectively. In alternative embodiments, the housing 940 may act as an active electrode in combination with the RV electrode 236 alone, or as part of a split electrical vector using the SVC coil electrode 238 (i.e., using the RV electrode as a common electrode). Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 9 to 40 joules), delivered asynchronously (since R-waves may be too disorganized), and pertaining exclusively to the treatment of fibrillation. Accordingly, the microcontroller 960 is capable of controlling the synchronous or asynchronous delivery of the shocking pulses.

In addition, the stimulation device may be configured to perform Automatic Mode Switching (AMS) wherein the pacemaker reverts from a tracking mode such as a VDD or DDD mode to a nontracking mode such as VVI or DDI mode. VDD, DDD, VVI and DDI are standard device codes that identify the mode of operation of the device. DDD indicates a device that senses and paces in both the atria and the ventricles and is capable of both triggering and inhibiting functions based upon events sensed in the atria and the ventricles. VDD indicates a device that sensed in both the atria and ventricles but only paces in the ventricles. A sensed event on the atrial channel triggers ventricular outputs after a programmable delay, the pacemaker equivalent of a PR interval. VVI indicates that the device is capable of pacing and sensing only in the ventricles and is only capable of inhibiting the functions based upon events sensed in the ventricles. DDI is identical to DDD except that the device is only capable of inhibiting functions based upon sensed events, rather than triggering functions. As such, the DDI mode is a non-tracking mode precluding its triggering ventricular outputs in response to sensed atrial events. Numerous other device modes of operation are possible, each represented by standard abbreviations of this type.

## Claims

1. A system for determining a preferred location for cardiac pacing for a patient, the system comprising: means for collecting information pertaining to myocardial activation times within a selected heart chamber of the patient; and means for determining the preferred location for pacing within the selected chamber by identifying the last activated site within the myocardium of the selected chamber.

2. A system as claimed in Claim 1 for determining a preferred location for delivering cardiac pacing pulses to the myocardium of a heart chamber of a patient, **characterized in that** the collecting means comprises circuitry operative to collect information for the patient pertaining to myocardial activation times within a selected heart chamber of the patient; and the determining means comprises an optimal pacing location determination system operative to determine a preferred location for pacing within the selected chamber based on the information pertaining to myocardial activation times by identifying the last activated site within the myocardium of the selected chamber.

3. A system as claimed in Claim 2, **characterized by** a mapping system operative to generate a map of the heart of the patient identifying the optimal pacing location.

4. A system as claimed in Claim 2 or Claim 3, **characterized by** a master pacemaker having endocardial electrodes implanted within selected chambers of the heart and a satellite pacemaker having an epicardial electrode mounted to a selected ventricle, with the epicardial electrode mounted at the preferred location.

5. A system as claimed in Claim 4, **characterized in that** the master pacemaker and the satellite pacemaker are configured to deliver cardiac resynchronization therapy via the respective endocardial and epicardial electrodes.

6. A system as claimed in any preceding Claim, **characterized by** a motion-sensing probe arranged to determine the myocardial activation times.

7. A system as claimed in Claim 6, **characterized in that** the motion sensing probe includes an accelerometer.

8. A system as claimed in any preceding Claim, **characterized by** tissue Doppler imaging means, arranged to determine the myocardial activation times

9. A system as claimed in any preceding Claim, **characterized by** an external programmer device arranged to determine the preferred location and also arranged to programme operations of implantable cardiac stimulation devices for implant within patients.
